# EUROPÄISCHE PATENTSCHRIFT

(11) **EP 1 243 257 B1**
(45) Veröffentlichungstag und Bekanntmachung des Hinweises auf die Patenterteilung: **28.01.2004**
(21) Anmeldenummer: 02005023.3
(22) Anmeldetag: 06.03.2002
(51) Int. Cl.: A61K 9/22, A61K 31/7036, A61L 31/16, A61L 27/54

(54) **Antibiotikum-/Antibiotika-Zubereitung mit retardierender Wirkstofffreisetzung**
Sustained release preparation comprising one or more antibiotics
Préparation à libération prolongée contenant un ou plusieurs antibiotiques

(30) Priorität: 22.03.2001 DE 10114244
(43) Veröffentlichungstag der Anmeldung: 25.09.2002
(73) Patentinhaber: Heraeus Kulzer GmbH & Co.KG, 63450 Hanau (DE)
(72) Erfinder: Vogt, Sebastian F., Dr., 07749 Jena (DE); Schnabelrauch, Matthias, Dr., 07745 Jena (DE); Kühn, Klaus-Dieter, Dr., 35041 Marburg (DE)
(74) Vertreter: Kühn, Hans-Christian

(56) Entgegenhaltungen:
- EP-A- 0 052 916
- DE-A- 2 802 273
- FR-A- 2 668 367
- GB-A- 1 120 992
- US-A- 4 291 013
- US-A- 5 035 891
- US-A- 5 670 142
- DATABASE WPI Derwent Publications Ltd., London, GB; AN 1995-373533 XP002200970 BELOV EV ET AL: "Haemostatic and antibacterial sponges for surgical use" & WO 95 27517 A (LYZION AUSTRALIA PTY LTD;ROSSIISKY NII GEMATOL (RU)), 19. Oktober 1995 (1995-10-19)

## Beschreibung

Die Erfindung betrifft eine Antibiotikum-/Antibiotika-Zusammensetzung und mehrere Verwendungen.

Die Behandlung lokaler mikrobieller Infektionen des Weich- und Hartgewebes in der Humanund Veterinärmedizin erfordert hohe lokale Antibiotika-Konzentrationen im infizierten Gewebebereich. Es ist seit langem bekannt, dass eine systemische Anwendung von Antibiotika mit einer Reihe von Problemen behaftet ist. Bei der systemischen Anwendung ist es oft erforderlich sehr hohe Antibiotika-Dosen einzusetzen, damit im infizierten Gewebe antimikrobiell wirksame Antibiotika-Konzentrationen erzielt werden. Dadurch kann es insbesondere bei den Aminoglucosid-Antibiotika und bei den Antibiotika vom Tetracyclin-Typ auf Grund ihrer Nephro- und Ototoxizität zu schwerwiegenden Schädigungen des Organismus kommen. Daher lag der Gedanke nahe, Antibiotika in lokal applizierbaren Freisetzungssystemen einzusetzen, beziehungsweise sie in geeignete Depotformen zu überführen.

Depotsysteme zur verzögerten Freisetzung von Antibiotika für die Behandlung von lokalen Infektionen sind Gegenstand einer Vielzahl von Veröffentlichungen und Patenten. Diese kann man allgemein nach zwei grundlegenden Retardierungsmechanismen einteilen. Das eine Wirkungsprinzip besteht in der physikalischen Fixierung der Antibiotika durch Adsorption an eine Matrix beziehungsweise durch Einschluß in eine nichtresorbierbare oder resorbierbare Matrix. Das zweite, chemische Verzögerungsprinzip besteht in der Verwendung von schwerlöslichen Antibiotika-Salzen, die sich nach entsprechender Applikation im humanen oder tierischen Organismus langsam unter Wirkstofffreisetzung auflösen.

Die physikalische Fixierung von Antibiotika unter Verwendung von nichtresorbierbaren Kunststoffen war Inhalt einer Reihe von Patenten, von denen hier nur einige exemplarisch aufgeführt werden. So schlägt Klemm (K. Klemm: Surgical synthetic-resin material and method of treating osteomyelitis. 13.05.1975, **US 3,882,858**) die Behandlung von Osteomyelitis mit Kunststoffpartikeln aus Polymethacrylat, Polyacrylat sowie deren Kopolymeren vor, die mit Gentamicin oder anderen Antibiotika beladen sind. Klemm beschreibt die Anwendung von Septopal (K. Klemm: Septopal - a new way of local antibiotic therapy. In T. J. G. Van Rens, F. H. Kayser (Eds.), Local antibiotic Treatment in Osteomyelitis and Soft-Tissue Infections, Excerpta Medica, Amsterdam (1981) 24-31; K. Klemm: Antibiotic beat chains. Clin. Orthop. Relat. Res. 295 (1993) 63-76.). Hierbei handelt es sich um kommerziell verfügbare Gentamycin-freisetzende Ketten aus Polymethacrylat. Heuser und Dingeldein beschreiben eine Komposition auf der Basis von Antibiotika und Polymethymethacrylat beziehungsweise Polyacrylat, der als zusätzliche Komponente Aminosäuren zugefügt sind (D. Heuser, E. Dingeldein: Synthetic resin-base, antibiotic compositions containing amino acids. 04.04.1980, **US 4,191,740**; D. Heuser, E. Dingeldein: Synthetic resin-base, antibiotic compositions containing amino acids. 11.11.1980, **US 4,233,287**). Weiterhin wurden auch Antibiotika, insbesondere Aminoglykosid-Antibiotika, in Knochenzemente integriert (A. Gross, R. Schaefer, S. Reiss: Bone cement compositions containing gentamycin. 22.11.1977, **US 4,059,684**; A. Welch: Antibiotics in acrylic bone cement. In vitro studies. J. Biomed. Mater. Res. 12 (1978) 679; R. A. Elson, A. E. Jephott, D. B. McGechie, D. Vereitas: Antibiotic-loaded acrylic cement. J. Bone Joint Surg. 59B (1977) 200-205.

Die physikalische Fixierung von Antibiotika mit Hilfe von resorbierbaren Kunststoffen, insbesondere von Polyestern der α-Hydroxycarbonsäuren, war ebenfalls Gegenstand einer Reihe von Publikationen, von denen hier ebenfalls nur einige exemplarisch referiert werden. Sampath et. al. schlagen ein Gentamicin-freisetzendes System bestehend aus Poly-L-lactid und Gentamicin vor, das durch Verpressung von Poly-L-lactid/Gentamicin-Mikrokapseln hergestellt wurde (S. S. Sampath, K. Garvin, D. H. Robinson: Preparation and characterization of biodegradable poly(-Llactic acid) gentamicin delivery systems. Int. J. Pharmaceutics 78 (1992) 165-174). Dieses System zeigt in Abhängigkeit von der eingesetzten Menge an Gentamicin eine nicht unbeträchtliche Verzögerung der Wirkstofffreisetzung. Bei einem ähnlichen System wurde Poly-D,Llactid zur Herstellung von wirkstoffenthaltenden Mikrosphären genutzt (R. Bodmeier, J. W. McGinity: The preparation and evaluation of drug-containing poly(D,L-lactide) microspheres formed by solvent evaporation method. Pharm. Res. 4 (1987) 465-471). Von Fries und Schlapp werden ebenfalls Mikropartikel aus Polylactid beschrieben, die mit Kollagen/Gentamicinsulfat beschichtet sind (W. Friess, M. Schlapp: Advanced implants for local delivery of gentamicin. Sixth World Biomaterials Congress Transactions (2000) 1488). Diese beschichteten Mikrosphären zeigten nur eine sehr geringe Tendenz, die Gentamicin-Freisetzung zu verzögern. Von Schmidt et al. wurden gentamicinenthaltende resorbierbare Formkörper vorgeschlagen (C. Schmidt, R. Wenz, B. Nies, F. Moll: Antibiotic in vivo/in vitro release, histocompatibility and biodegradation of gentamicin implants based on lactic acid polymers and copolymers. J. Control. Release 37 (1995) 83-94). Diese Körper wurden durch Verpressung von Gemischen aus Gentamicin-Sulfat/Poly-L-lactid, Gentamicin-Sulfat/Poly-D,L-lactid und Gentamicinsulfat/Poly-D,Llactid-co-glykolid hergestellt. Diese Depotpräparate setzten zirka neunzig Prozent des Antibiotikums innerhalb von vierundzwanzig Stunden frei.

Neben der physikalischen Fixierung von Antibiotika unter Verwendung von Kunststoffen wurden auch zahlreiche anorganische Systeme mit retardierender Wirkung beschrieben. Im folgenden werden exemplarisch nur einige der mit Calciumsulfat hergestellten Systeme kurz referiert. So wird von Randolph et al. ein retardierendes System beschrieben, dass auf dem Einschluß von Wirkstoffen in eine Calciumsulfat-Matrix beruht (D. A. Randolph, J. L. Negri, T. R. Devine, S. Gitelis: Calcium sulfate controlled release matrix. 15.09.1998, **US 5,807,567**). Die Herstellung dieser Calciumsulfat-Pellets erfolgt dabei ausgehend von einem Gemisch aus α-Calciumsulfat-Hemihydrat, β-Calciumsulfat-Hemihydrat, einem Additiv und Wasser. Die Härtung erfolgt durch Bildung von Calciumsulfat-Dihydrat. Turner et al. beschreiben Tabletten aus Calciumsulfat, die Tobramycin enthalten und zur Behandlung von Medullar-Defekten Verwendung finden sollen (T. M. Turner, R. M. Urban, S. Gitelis, A. M. Lawrence-Smith, D. J. Hall: Delivery of tobramycin using calcium sulfate tablets to graft a large medullary defect: Local and systemic effects. Sixth World Biomaterials Congress Transactions (2000) 767.) Ähnliche Freisetzungssysteme aus Calciumsulfat, aber mit Amikacin-Sulfat, werden ebenfalls beschrieben (D. W. Petersen, W.O. Haggard, L. H. Morris, K. C. Richelsoph, J. E. Parr: Elution of amikacin from calcium sulfate pellets: An in vitro study. Sixth World Biomaterials Congress Transactions (2000) 767).

Bisher fanden schwerlösliche Salze der Aminoglykosid-Antibiotika, der Tetracyclin-Antibiotika und der Lincosamid-Antibiotika relativ geringe Beachtung für die Herstellung von Depotpräparaten. Die Bildung von schwerlöslichen Salzen beziehungsweise Chelaten der Antibiotika des Tetracyclintyps ist seit Jahrzehnten allgemeiner Kenntnisstand. So beschreibt Folch Vazquez die Herstellung von Tetracyclindodecylsulfat durch Umsetzung von Tetracyclinhydrochlorid mit Natriumdodecylsulfat in Wasser (C. Folch Vazquez: Tetracycline lauryl sulfate. 08.02.1966, **ES 3 309 402;** C. Folch Vazquez: Tetracycline derivatives. 09.01.1967, **NL 6609490)** Alternativ kann die Herstellung auch ausgehend von Tetracyclin und der Dodecylschwefelsäure erfolgen (C. Folch Vazquez: Tetracycline lauryl sulfate. 08.02.1966, **ES 322 771).** Weiterhin wurde auch die Verwendung von Tetracylin-Sulfamaten zur antibiotischen Therapie vorgeschlagen (A. Jurando, J. M. Puigmarti: Antibiotic tetracycline sulfamate and its derivatives. 27.10.1970, **US 3,536,759**; Anonym: Antibiotic tetracycline alkylsulfamates. 16.10.1969, **ES 354 173;** C. Ciuro, A. Jurado: Stability of a tetracycline derivative. Afinidad 28 (292) 1971, 1333-5.). Bei den Aminoglykosid-Antibiotika sind ebenfalls eine Reihe von schwerlöslichen Salzen prinzipiell bekannt. So wurde beim Gentamicin die Darstellung schwerlöslicher Salze basierend auf höheren Fettsäuren, Arylalkylcarbonsäuren, Alkylsulfaten und Alkylsulfonaten beschrieben ( G. M. Luedemann, M. J. Weinstein: Gentamycin and method of production. 16.07.1962, **US 3,091,572** ). Exemplarisch sind dafür Gentamicin-Salze der Laurinsäure, der Stearinsäure, der Palmitinsäure, der Ölsäure, der Phenylbuttersäure, der Naphthalen-1-carbonsäure, der Laurylschwefelsäure und der Dodecylbenzensulfonsäure. Diese Salze erwiesen sich vielfach als unvorteilhaft, weil sie wachsartige, hydrophobe Substanzen darstellen, die eine galenische Verwendung behindern. Trotzdem wurden Festtsäuresalze von Gentamicin und von Etamycin aus der freien Base beziehungsweise aus ihren Salzen in Wasser bei 50-80°C synthetisiert (H. Voege, P. Stadler, H. J. Zeiler, S. Samaan, K. G. Metzger: Sparingly-soluble salts of aminoglycosides and formations containing them with inhibited substance-release. 28.12.1982 **DE 3 248 328**). Diese Antibiotika-Fettsäuresalze sollen als Injektionspräparate geeignet sein. Die Herstellung von Gentamicindodecylsulfat und dessen Verwendung in Salben, Cremes wurde ebenfalls beschrieben (C. Folch Vazquez: Gentamicin derivates. 29.10.1974, **BE 821 600**). Auch bei den Lincosamid-Antibiotika sind schwerlösliche Salze, wie zum Beispiel das Glindamycin-Palmitat bekannt (M. Cimbollek, B.Nies, R. Wenz, J. Kreuter: Antibiotic-impregnated heart valve sewing rings for treatment and prophylaxis of bacterial endocarditis. Antimicrob. Agents Chemother. 40(6) (1996)1432-1437). Eine neuere Entwicklung stellen schwerlösliche Aminoglykosid-Flavonoid-Phosphate dar (H. Wahlig, E. Dingeldein, R. Kirchlechner, D. Orth, W. Rogalski: Flavonoid phosphate salts of aminoglycoside antibiotics. 13.10.1986, **US 4,617,293**). Es werden die Salze der Phosphorsäurehalbester von Derivaten der Hydroxyflavane, Hydroxyflavene, Hydroxyflavanone, Hydroxyflavone und Hydroxyflavylium beschrieben. Besonders bevorzugt sind die Derivate der Flavanone und der Flavone. Diese schwerlöslichen Salze sollen als Depotpräparate Verwendung finden. So werden zum Beispiel diese Salze in Kollagenvliese eingebracht (H. Wahlig, E. Dingeldein, D. Braun: Medicinally useful, shaped mass of collagen resorbable in the body. 22.09.1981, **US 4,291,013**). Weiterhin wurden auch künstliche Herzklappen mit diesen schwerlöslichen Gentamicin-Salzen, Gentamicin-Crobefat, imprägniert (M. Cimbollek, B.Nies, R. Wenz, J. Kreuter: Antibiotic-impregnated heart valve sewing rings for treatment and prophylaxis of bacterial endocarditis. Antimicrob. Agents Chemother. 40(6) (1996)1432-1437). Interessant ist bei diesem Patent insbesondere, dass ein Gemisch aus leichtlöslichem Gentamicin-Sulfat und schwerlöslichem Gentamicin-Crobefat eingesetzt wird. Das Ziel war dabei, dass einerseits nach Einbringung der Herzklappenringe in den Organismus beziehungsweise in eine Modellflüssigkeit eine hohe initiale Gentamicin-Konzentration durch das leichtlösliche Gentamicin-Sulfat erreicht wird und das andererseits durch das relativ schwerlösliche Gentamicin-Crobefat eine Freisetzung von Gentamicin über einen längeren Zeitraum möglich wird. Das bedeutet, die zeitabhängige Freisetzung des Gentamicins wird durch das Verhältnis aus leichtlöslichem Gentamicin-Sulfat und schwerlöslichem Gentamicin-Crobefat gesteuert. Für eine gezielte Einstellung des Freisetzungsverhaltens ist es daher erforderlich, die beiden Gentamicin-Salze in definierten Mengenverhältnissen in die galenischen Formulierungen einzubringen. Diese Methode der Depotbildung durch Kombination eines leichtlöslichen Antibiotika-Salzes mit einem schwerlöslichen Antibiotika-Salz setzt die Verfügbarkeit einer reinen schwerlöslichen Salzform eines Antibiotikums voraus.

Zusammenfassend kann festgestellt werden, dass die bekannten Antibiotika-Depotsysteme mit physikalisch verursachter Verzögerung der Antibiotika-Freisetzung in starkem Maße von der Zusammensetzung und Struktur der verwendeten Matrix abhängen. Weiterhin ist der Herstellungsprozeß dieser Antibiotika-Depotsysteme von nicht unerheblichem Einfluß auf das Freisetzungsverhalten. Der Nachteil von Systemen mit schwerlöslichen Antibiotika-Salzen besteht darin, dass für jedes verwendete Antibiotikum eine spezielle Salzform vor der Herstellung der Depotpräparate synthetisiert werden muss.

Der vorliegenden Erfindung liegt das Problem zugrunde, eine Antibiotikum-/Antibiotika-Zubereitung mit retardierender Wirkstoffreisetzung als resorbierbare und auch nichtresorbierbare Implantate im Bereich der Human- und Veterinärmedizin, zur Behandlung lokaler mikrobieller Infektionen im Knochen- und im Weichgewebe zu entwickeln, die die Nachteile der bekannten retardierenden Antibiotika-Formulierungen überwindet. Angestrebt wird eine Antibiotikum-/Antibiotika-Zubereitung, die eine kontrollierte Antibiotika-Freisetzung über einen Zeitraum bis ungefähr drei Wochen ermöglicht. Der Mechanismus der verzögerten Wirkstofffreisetzung sollte im wesentlichen unabhängig von Trägermaterialien sein und nicht auf Adsorptionseffekten an den Oberflächen von Trägermaterialien beruhen. Angestrebt wird eine Antibiotikum-/Antibiotika-Zubereitung, die unter Beibehaltung der Wirkstoffretardierung sowohl mit resorbierbaren als auch nichtresorbierbaren Hilfsstoffen unterschiedlichster Struktur zu Implantaten verarbeitet werden kann. Weiterhin soll die Art und Weise der Antibiotikum-/Antibiotika-Zubereitung nicht nur für ein spezielles Antibiotikum anwendbar sein, sondern es sollte vielmehr für eine Reihe Antibiotika ähnlicher Struktur geeignet sein.

Dieses Problem wird erfindungsgemäß durch eine Antiobiotika-Zubereitung nach **Anspruch 1, Verwendungen nach den Ansprüchen 7-13, und ein Verfahren nach Anspruch 14 gelöst.**

Der Erfindung liegt der überraschende Befund zu Grunde, dass ein Gemisch aus mindestens einer amphiphilen Komponente eines Vertreters der Alkylsulfate, Arylsulfate, Alkylarylsulfate, Cycloalkylsulfate, Alkylcycloalkylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Arylsulfonate, Alkylarylsulfonate, Cycloalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate sowie mindestens einer antibiotischen Komponente aus der Gruppe der Aminoglykosid-Antibiotika, der Lincosamid-Antibiotika und Tetracyclin-Antibiotika eine retardierende Wirkstoffreisetzung über einen Zeitraum von mehreren Tagen bis zu mehreren Wochen im wässrigen Milieu zeigt.

Die nachfolgenden Ausführungsformen haben sich in der Praxis als besonders vorteilhaft bewährt.

Zunächst ist es von Vorteil, dass die Antibiotika-Zubereitung mindestens eine wasserfreie, organische Hilfskomponente aufweist, die hydrolytisch spaltbare Carbonsäureesterbindungen und/oder hydrolytisch spaltbare Carbonsäureamidbindungen und/oder hydrolytisch spaltbare Carbonsäureanhydridbindungen und/oder hydrolytisch spaltbare Phosphorsäuresterbindungen und/oder hydrolytisch spaltbare Phosphorsäureamidbindungen und/oder enzymatisch spaltbare Carbonsäureesterbindungen und/oder enzymatisch spaltbare Carbonsäureamidbindungen und/oder enzymatisch spaltbare Carbonsäureanhydridbindungen und/oder enzymatisch spaltbare Phosphorsäuresterbindungen und/oder enzymatisch spaltbare Phosphorsäureamidbindungen aufweist.

Weiterhin ist es von Vorteil, wenn die Antibiotika-Zubereitung mindestens eine anorganische Hilfskomponente aus der Gruppe Calciumhydrogenphosphat, Calciumhydrogenphosphat-Dihydrat, Hydroxylapatit, Fluorapatit, Calciumpolyphosphat, Tricalciumphosphat, Tetracalciumphosphat, Calciumsulfat, Calciumsulfat-Hemihydrat, Calciumsulfat-Dihydrat, Calciumlactat, Natriumhydrogencarbonat, Calciumcarbonat, Magnesiumcarbonat, Calciumhydroxid, Magnesiumhydroxid, Magnesiumoxid - vorangehende Stoffe in Form grobdisperser und/oder hochdisperser Pulver -, resorbierbare Gläser, nichresorbierbare Gläser, resorbierbare Glaskeramik, nichtresorbierbare Glaskeramik, resorbierbare Keramik und nichtresorbierbare Keramik enthält.

Darüber hinaus ist es vorteilhaft, wenn die Antibiotika-Zubereitung mindestens eine biologisch aktive Hilfskomponente aus der Gruppe der Penicilin-Antibiotika, der Cephalosporin-Antibiotika, der 4-Chinolon-Antibiotika und der Makrolid-Antibiotika oder gegebenenfalls ein oder mehrere Vertreter der Sulfonamid-Chemotherapeutika, der Analgetika und der Antiphlogistika enthält.

Erfindungsgemäß ist es von Vorteil, dass die amphiphile Komponente aus der Gruppe der Alkylsulfate, Arylsulfate, Alkylarylsulfate, Cycloalkylsulfate und Alkylcycloalkylsulfate als Halbester in der Natriumsalzform und/oder in der Kaliumsalzform und/oder in der Ammoniumsalzform und/oder in der Trialkylammoniumsalzform und/oder in der Dialkylammoniumsalzform und/oder der Monoalkylammoniumsalzform und/oder in der Triarylammoniumsalzform und/oder in der Diarylammoniumsalzform und/oder in der Arylammoniumsalzform und/oder in der Alkyldiarylammoniumsalzform und/oder in der Dialkylarylammoniumsalzform und/oder der Tricycloalkylammoniumsalzform und/oder der Dicycloalkylammoniumsalzform und/oder der Monocycloalkylammoniumsalzform und/oder der Alkyldicycloalkylammoniumsalzform und/oder in der Dialkylcycloalkylammoniumsalzform und/oder in der Säureform und/oder in der Anhydridform vorliegt.

Weiterhin ist es erfindungsgemäß vorteilhaft, dass die amphiphile Komponente aus der Gruppe der Alkylsulfonate, Fettsäure-2-sulfonate, Alkylsulfamate, Cycloalkylsulfamate, Arylsulfamate, Alkylarylsulfamate, Arylsulfonate, Alkylarylsulfonate, Cycloaalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate in der Natriumsalzform und/oder in der Kaliumsalzform und/oder in der Ammoniumsalzform und/oder in der Trialkylammoniumsalzform und/oder in der Dialkylammoniumsalzform und/oder der Monoalkylammoniumsalzform und/oder in der Triarylammoniumsalzform und/oder in der Diarylammoniumsalzform und/oder in der Arylammoniumsalzform und/oder in der Alkyldiarylammoniumsalzform und/oder in der Dialkylarylammoniumsalzform und/oder der Tricycloalkylammoniumsalzform und/oder der Dicycloalkylammoniumsalzform und/oder der Monocycloalkylammoniumsalzform und/oder der Alkyldicycloalkylammoniumsalzform und/oder in der Dialkylcycloalkylammoniumsalzform und/oder in der Sulfonsäureform und/oder in der Sulfonsäureanhydridform vorliegt.

Erfindungsgemäß ist es auch von Vorteil, dass die antibiotische Komponente mindestens eine Aminogruppe enthält.

Ferner ist es erfindungsgemäß vorteilhaft, dass als amphiphile Komponente mindestens eine Verbindung aus der Gruppe der Alkylsulfate, Cycloalkylsulfate, Cycloalkylalkylsulfate, Arylsulfate, Alkylarylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Cycloalkylsulfonate, Cycloalkylalkylsulfonate, Arylsulfonate, und Alkylarylsulfonate mit jeweils 6 bis 30 Kohlenstoffatomen bevorzugt sind.

Erfindungsgemäß ist, dass aus monocyclischen, dicyclischen, tricyclischen, tetracyclischen, pentacyclischen, hexacyclischen, heptacyclischen und octacyclischen aromatischen Ringsystemen aufgebaute Arylsulfate, Alkylarylsulfate, Arylsulfamate, Alkylarylsulfamate, Aryldisulfonate, Alkylaryldisulfonate, Aryltrisulfonate und Alkylaryltrisulfonate als amphiphile Komponente bevorzugt sind.

Erfindungsgemäß ist, dass aus monocyclischen, dicyclischen, tricyclischen, tetracyclischen, pentacyclischen, hexacyclischen, heptacyclischen und octacyclisachen gesättigten Ringsystemen aufgebaute Cycloalkylsulfate, Alkylcycloalkylsulfate, Cycloalkylsulfonate, Alkylcycloalkylsulfonate, Cycloalkylsulfamate, und Alkylcycloalkylsulfamate als amphiphile Komponente bevorzugt sind.

Erfindungsgemäß ist, dass Natriumdodecylsulfat, Natriumtetradecylsulfat, Natriumhexadecylsulfat, Natriumoctadecylsulfat, Natriumdocosanylsulfat, Natriumdodecylsulfonat, Natriumtetradecylsulfonat, Natriumhexadecylsulfonat, Natriumoctadecylsulfonat, Natriumdodecylbenzylsulfonat und Natriumcholesterolsulfat als amphiphile Komponente besonders bevorzugt werden.

Weiterhin ist erfindungsgemäß, dass als antibiotische Komponente besonders Allomycin, Amicetin, Amikacin, Apramycin, Bekanamycin, Betamicin, Butirosin, Destomycin, Dibekacin, Dihydrostreptomycin, Flambamycin, Fortimycin A, Fortimycin B, Framycetin, Gentamicin, Hikizimycin, Homomycin, Hybrimycin, Hygromycin B, Kanamycin, Kasuhamycin, Lividomycin, Minosaminoycin, Neomycin, Netilmicin, Paromomycin, Parvulomycin, Puromycin A, Ribostamycin, Rimocidin, Ristosamin, Ristomycin, Sagamycin, Sisomicin, Sorbistin, Spectinomycin, Streptomycin, Tobramycin, Tunicamycin, Vancomycin, Verdamycin aus der Gruppe der Aminoglykosid-Antibiotika bevorzugt werden.

Erfindungsgemäß ist, dass als antibiotische Komponente Clindamycin und Lincomycin aus der Gruppe der Lincosamid-Antibiotika bevorzugt werden.

Ferner wird als antibiotische Komponente Ciprofloxacin oder Moxifloxacin aus der Gruppe der 4-Chinolon-Antibiotika bevorzugt verwendet.

Erfindungsgemäß ist, dass als antibiotische Komponente Tetracyclin, Chlortetracyclin, Oxytetracylin, Demethylchlortetracyclin, Methacyclin, Doxycyclin, Rolitetracyclin und Minocyclin aus der Gruppe der Tetracyclin-Antibiotika bevorzugt werden.

Es ist auch erfindungsgemäß, dass die antibiotische Komponente in der protonierten Salzform vorliegt, wobei Chlorid-Ionen, Bromid-Ionen, Hydrogensulfat-Ionen, Sulfat-Ionen, Dihydrogenphosphat-Ionen, Hydrogenphosphat-Ionen, Phosphat-Ionen, Acetat-Ionen, Succinat-Ionen und Lactat-Ionen als Gegenionen verwendet werden.

Erfindungsgemäß ist weiterhin, dass bevorzugt 0,01 bis 10 Molmengenteile der amphiphilen Komponente mit einem Molmengenteil der antibiotischen Komponente vermischt sind.

Es ist erfindungswesentlich, dass durch das Verhältnis der Stoffmenge der amphiphilen Komponente zur Stoffmenge der antibiotischen Komponente der Anteil der verzögert freigesetzten antibiotischen Komponente an der Gesamtmenge der antibiotischen Komponente bestimmt werden kann.

Es ist auch vorteilhafterweise erfindungsgemäß, dass mindestens eine Verbindung aus der Gruppe Oligoester und Polyester der L-Milchsäure und/oder der D-Milchsäure und/oder der 2-Hydroxyethansäure und/oder der 2-Hydroxyethoxyethansäure und/oder der 3-Hydroxybutansäure und/oder der 4-Hydroxybutansäure und/oder der 4-Hydroxyhexansäure und/oder der 6-Hydroxyhexansäure und gegebenenfalls Kooligoester und/oder Kopolyester und ggf. Teroligoester und/oder Terpolyester dieser Hydroxycarbonsäuren als wasserfreie, organische Hilfskomponente verwendet werden.

Es ist erfindungsgemäß, dass Oligoamide und/oder Polyamide als wasserfreie, organische Hilfskomponente verwendet werden, die als Bestandteile Aminosäuren enthalten.

Erfindungsgemäß ist, dass die Aminosäuren Glycin und/oder L-Alanin und/oder D-Alanin und/oder L-Valin und/oder D-Valin und/oder L-Threonin und/oder D-Threonin und/oder L-Asparinsäure und/oder D-Asparaginsäure und/oder L-Asparagin und/oder D-Asparagin und/oder L-Glutaminsäure und/oder D-Glutaminsäure und/oder L-Glutamin und/oder D-Glutamin und/oder L-Ornithin und/oder D-Ornithin und/oder L-Lysin und/oder D-Lysin und/oder 3-Aminopropansäure und/oder R-2-Aminobutansäure und S-2-Aminobutansäure und/oder 3-Aminobutansäure und/oder 4-Aminobutansäure und/oder R-2-Aminopentansäure und/oder S-2-Aminobutansäure und/oder 3-Aminopentansäure und/oder 4-Aminopentansäure und/oder 5-Aminopentansäure und/oder R-2-Aminohexansäure und/oder S-2-Aminohexansäure und/oder 3-Aminohexansäure und/oder 4-Aminohexansäure und/oder 5-Aminohexansäure und/oder 6-Aminohexansäure und/oder R-2-Aminoheptansäure und/oder S-2-Aminoheptansäure und/oder 3-Aminoheptansäure und/oder 4-Aminoheptansäure und/oder 5-Aminoheptansäure und/oder 6-Aminoheptansäure und/oder 7-Aminoheptansäure und/oder R-2-Aminooctansäure und/oder S-Aminooctansäure und/oder 3-Aminooctansäure und/oder 4-Aminooctansäure und/oder 5-Aminooctansäure und 6-Aminooctansäure und/oder 7-Aminooctansäure und/oder 8-Aminooctansäure und/oder R-2-Aminononansäure und/oder S-2-Aminononansäure und/oder 3-Aminononansäure und/oder 4-Aminononansäure und/oder 5-Aminononansäure und/oder 6-Aminononansäure und/oder 7-Aminononansäure und/oder 8-Aminononansäure und/oder 9-Aminononansäure und/oder R-2-Aminodecansäure und/oder S-2-Aminodecansäure und/oder 3-Aminodecansäure und/oder 4-Aminodecansäure und/oder 5-Aminodecansäure und/oder 6-Aminodecansäure und/oder 7-Aminodecansäure und/oder 8-Aminodecansäure und/oder 9-Aminodecansäure und/oder 10-Aminodecansäure und/oder der 11-Aminoundecansäure und/oder L-Phenylalanin und/oder D-Phenylalanin und/oder L-Tyrosin und/oder D-Tyrosin und/oder L-Histidin und/oder D-Histidin und/oder L-Tryptophan und/oder D-Tryptophan als Bausteine der Oligoamide und Polyamide verwendet werden.

Erfindungsgemäß ist, dass bevorzugt aliphatische Alkohole mit einer Anzahl von 12 bis 30 Kohlenstoffatomen als wasserfreie, organische Hilfskomponente verwendet werden.

Ferner ist erfindungsgemäß, dass bevorzugt Fettsäuren mit einer Anzahl von 12 bis 30 Kohlenstoffatomen als wasserfreie, organische Hilfskomponente verwendet werden.

Erfindungsgemäß ist auch, dass Glycerintrifettsäureester, Glycerindifettsäureester und Glycerinmonofettsäureester als wasserfreie, organische Hilfskomponente bevorzugt sind, wobei die Fettsäurereste jeweils 14 bis 22 Kohlenstoffatome enthalten.

Erfindungsgemäß ist, dass n-Alkane und iso-Alkane mit 6 bis 30 Kohlenstoffatomen als wasserfreie, organische Hilfskomponente bevorzugt sind.

Erfindungsgemäß ist, dass Polyethylenglykol und/oder Polypropylenglykol mit Molmassen im Bereich von 200 bis 35000 als wasserfreie, organische Hilfskomponente bevorzugt sind.

Erfindungsgemäß ist, dass Polyethylenoxid und/oder Polypropylenoxid mit Molmassen im Bereich von 35000 bis 1000000 als wasserfreie, organische Hilfskomponente bevorzugt sind.

Erfindungsgemäß ist, dass als wasserfreie, organische Hilfskomponente mindestens eine Verbindung aus der Gruppe Gelatine, Kollagen, Cellulose, Carboxymethylcellulose, Methylcellulose, Ethylcellulose. Hydroxyethylcellulose, Propylcellulose, Hydroxypropylcellulose, Butylcellulose, Stärke, Carboxymethylstärke, Methylstärke, Ethylstärke, Hydroxyethylstärke, Propylstärke, Hydroxypropylstärke, Butylstärke, Chitin Carboxymethylchitin, Chitosan, Carboxymethylchitosan, Glykogen, Carboxymethylglykogen, Alginsäure, Alginsäuremethylether, Hyaluronsäure, Carboxymethylhyaluronsäure, Celluloseacetat, Cellulosepropionat, Cellulosebutyrat, Cellulosephthalat, Cellulosesulfat, Cellulosephosphat, Stärkeacetat, Stärkepropionat, Stärkebutyrat, Stärkephthalat, Stärkesulfat, Stärkephosphat, oxidierte Cellulose, oxidierte Stärke, Pullulan, Araban, Xanthan und Guar Gum bevorzugt ist.

Erfindungsgemäß ist, dass als wasserfreie, organische Hilfskomponente Carnaubawachs, Bienenwachs, Benzoeharz, Kollophonium und Kopalharz bevorzugt sind.

Erfindungsgemäß ist, dass als wasserfreie, organische Hilfskomponente mindestens eine Verbindung aus der Gruppe Polyethylen, Polypropylen, Polybutadien, Polyisopren, Polychlorbutadien, Polymethylmethacrylat, Poly-2-hydroxyethylmethacrylat, Polymethylacrylat, Polystyrol, Polyvinylacetat, Polyvinylalkohol, Polyvinylchlorid, Polyvinylidenchlorid, Polyvinylfluorid, Polyvinylpyrrolidon, Polytetrafluorethylen, Polycarbonat, Polysulfon, Polysiloxan und Gemische dieser Polymere bevorzugt ist.

Erfindungsgemäß ist, dass als wasserfreie, organische Hilfskomponente mindestens eine Verbindung aus der Gruppe Acrylsäurester, Acrylsäureamide, Methacrylsäurester, Methacrylsäureamide, Itaconsäureester, Maleinimide und deren Gemische bevorzugt ist.

Erfindungsgemäß ist es vorteilhaft, dass die wasserfreie, organische Hilfskomponente in festem und/oder in flüssigem Aggregatzustand vorliegt.

Auch erfindungsgemäß ist, dass die Arylsulfate, Arylsulfonate, Arylsulfamate und Alkylarylsulfonate gegebenenfalls Bestandteile eines nichtvernetzten und/oder eines vernetzten Polymers sind, wobei Polymere aus der Gruppe der Polystyrole, der Polymethacrylate, Polyacrylate, Polyamide, der Polycarbonate und/oder deren Kopolymere und/oder deren Terpolymere bevorzugt sind.

Darüber hinaus ist es von Vorteil, wenn die Antibiotika-Zusammensetzung als durch Pressen und/oder Strangpressen und/oder Mahlen und/oder Kalandrieren und/oder Gießen und/oder Spinnen und /der Sintern hergestellte Formkörper, Granulate, Folien, Pulver, Schläuche, Vliese oder Fäden vorliegen.

Darüber hinaus ist es von Vorteil, dass die salzartige Komponente und die antibiotische Komponente in der wasserfreien, organischen Hilfskomponente suspendiert sind und eine injizierbare Suspension bilden.

Schließlich ist es von besonderer Bedeutung, dass die erfindungsgemäße Antibiotika-Zubereitung als Implantate in Form von Formkörpern, Granulaten, Pulvern, Schläuchen, Folien, Vliesen und Fäden, insbesondere, wenn diese plastisch verformbar und modellierbar sind, verwendet werden können. Dies gilt auch für mögliche Beschichtungen auf resorbierbaren porösen Gläsern, auf nicht resorbierbare Gläsern, resorbierbaren porösen Glaskeramiken, nicht resorbierbaren porösen Glaskeramiken, resorbierbaren porösen Keramiken und nicht resorbierbaren porösen Keramiken inklusive resorbierbaren Kunststoffimplantaten, nicht resorbierbaren Kunststoffimplantaten und Metallimplantaten.

Durch das Verhältnis der Stoffmenge der amphiphilen Komponente zur Stoffmenge der antibiotischen Komponente kann der Anteil der verzögert freigesetzten antibiotischen Komponente an der Gesamtmenge der antibiotischen Komponente bestimmt werden.

Der Gegenstand der vorliegenden Erfindung soll anhand der nachfolgenden Beispiele 1-6 näher erläutert werden.

Herstellung der Antibiotikum-/Antibiotika-Zubereitungen

### Beispiel 1:

Es wird ein Gemisch aus 51 mg Gentamicinsulfat (700 U/mg, Fluka), 51 mg Natriumdodecylsulfat (Aldrich), 280 mg Poly-L-Lactid (Molmasse ~10.000 gmol⁻¹) und 1118 mg Calciumhydrogenphosphat (Fluka) hergestellt. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepreßt.

### Beispiel 2

Es wird ein Gemisch aus 51 mg Gentamicinsulfat (700 U/mg, Fluka), 51 mg Natriumdodecylsulfat (Aldrich), 280 mg Poly-L-Lactid (Molmasse ~10.000 gmol⁻¹) und 1118 mg Calciumhydrogenphosphat-Dihydrat (Fluka) hergestellt. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepreßt.

### Beispiel 3

Es wird ein Gemisch aus 51 mg Gentamicinsulfat (700 U/mg, Fluka), 51 mg Natriumdodecylsulfat (Aldrich), 280 mg Poly-L-Lactid (Molmasse ∼10.000 gmol⁻¹) und 1118 mg Calciumsulfat-Dihydrat (Fluka) hergestellt. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepreßt.

### Beispiel 4

Es wird ein Gemisch aus 51 mg Gentamicinsulfat (700 U/mg, Fluka), 51 mg Natriumdodecylsulfat (Aldrich), 280 mg Carnaubawachs (Aldrich) und 1118 mg Calciumhydrogenphosphat (Fluka) hergestellt. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepreßt.

### Beispiel 5

Es wird ein Gemisch aus 51 mg Gentamicinsulfat (700 U/mg, Fluka), 51 mg Natriumdodecylsulfonat (Fluka), 280 mg Poly-L-Lactid (Molmasse ∼10.000 gmol⁻¹) und 1118 mg Calciumhydrogenphosphat (Fluka) hergestellt. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepreßt.

### Beispiel 6

Es wird ein Gemisch aus 51 mg Gentamicinsulfat (700 U/mg, Fluka), 51 mg Natriumdodecylbenzylsulfonat (Fluka), 280 mg Poly-L-Lactid (Molmasse ~10.000 gmol⁻¹) und 1118 mg Calciumhydrogenphosphat (Fluka) hergestellt. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepreßt.

### Antibiotika-Freisetzungsversuche

Die in den Beispielen 1-6 hergestellten Formkörper wurden in physiologische Kochsalzlösung eingebracht und in dieser bei 37°C über einen Zeitraum von vier Wochen gelagert, um die retardierte Antibiotika-Freisetzung zu bestimmen. Die Probennahme erfolgte nach 1,3,6,9,12,14 und 21 Tagen Lagerungszeit. Die Antibiotika-Wertbestimmung wurde mit einem Agardiffusionstest unter Verwendung von Bacillus subtilis ATCC 6633 als Testkeim durchgeführt (Ergebnisse siehe **Tab.1**).

**Tab.1:**

| Kumulierte Gentamicin-Freisetzung aus Probekörpern der **Beispiele 1-6** in Abhängigkeit von der Lagerungszeit in physiologischer Kochsalzlösung bei 37°C. | | | | | | | | |
|---|---|---|---|---|---|---|---|---|
| Beispiele | kumulierte Gentamicin-Freisetzung [Ma%] | | | | | | | |
| | Lagerungszeit [d] | | | | | | | |
| | 1 | 3 | 6 | 9 | 12 | 14 | 21 | 51 |
| **1** | 32 | 54 | 67 | 72 | 77 | 83 | 94 | 100 |
| **2** | 45 | 54 | 63 | 71 | 77 | 82 | 88 | 100 |
| **3** | 48 | 57 | 64 | 78 | 84 | 91 | 100 | 100 |
| **4** | 43 | 51 | 58 | 71 | 81 | 93 | 100 | 100 |
| **5** | 50 | 69 | 85 | 95 | 99 | 100 | 100 | 100 |
| **6** | 77 | 82 | 86 | 90 | 94 | 97 | 100 | 100 |

## Patentansprüche

1. Antibiotikum-/Antibiotika-Zubereitung, **gekennzeichnet durch** ein Gemisch aus
- mindestens einer amphiphilen Komponente eines Vertreters der Alkylsulfate, Arylsulfate, Alkylarylsulfate, Cycloalkylsulfate, Alkylcycloalkylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Arylsulfonate, Alkylarylsulfonate, Cycloalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate
- mindestens einer antibiotischen Komponente aus der Gruppe der Aminoglykosid-Antibiotika, der Lincosamid-Antibiotika, der 4-Chinolon-Antibiotika und der Tetracyclin-Antibiotika sowie
- a) mindestens einer anorganischen Hilfskomponente aus den Gruppen
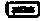 Calciumhydrogenphosphat, Calciumhydrogenphosphat-Dihydrat, Hydroxylapatit, Fluorapatit, Calciumpolyphosphat, Tricalciumphosphat, Tetracalciumphosphat, Calciumsulfat, Calciumsulfat-Hemihydrat, Calciumsulfat-Dihydrat, Calciumlactat, Natriumhydrogencarbonat, Calciumcarbonat, Magnesiumcarbonat, Calciumhydroxid, Magnesiumhydroxid, Magnesiumoxid
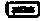 vorangehende Stoffe in Form grobdisperser und/oder hochdisperser Pulver,
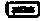 resorbierbare Gläser, nichresorbierbare Gläser, resorbierbare Glaskeramik, nichtresorbierbare Glaskeramik, resorbierbare Keramik und nichtresorbierbare Keramik und
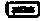 b) mindestens einer wasserfreien, organischen Hilfskomponente, die hydrolytisch spaltbare Carbonsäureesterbindungen und/oder hydrolytisch spaltbare Carbonsäureamidbindungen und/oder hydrolytisch spaltbare Carbonsäureanhydridbindungen und/oder hydrolytisch spaltbare Phosphorsäuresterbindungen und/oder hydrolytisch spaltbare Phosphorsäureamidbindungen und/oder enzymatisch spaltbare Carbonsäureesterbindungen und/oder enzymatisch spaltbare Carbonsäureamidbindungen und/oder enzymatisch spaltbare Carbonsäureanhydridbindungen und/oder enzymatisch spaltbare Phosphorsäuresterbindungen und/oder enzymatisch spaltbare Phosphorsäureamidbindungen aufweist.

2. Antibiotikum-/Antibiotika-Zubereitung nach Anspruch **1**, **dadurch gekennzeichnet, dass** diese mindestens eine biologisch aktive Hilfskomponente aus der Gruppe der Penicilin-Antibiotika, der Cephalosporin-Antibiotika, der 4-Chinolon-Antibiotika und der Makrolid-Antibiotika oder gegebenenfalls ein oder mehrere Vertreter der Sulfonamid-Chemotherapeutika, der Analgetika und der Antiphlogistika enthält.

3. Antitbiotikum-/Antibiotika-Zubereitung nach einem der Ansprüchen 1 bis **2**, **dadurch gekennzeichnet, dass** die antibiotische Komponente mindestens eine Aminogruppe enthält.

4. Antibiotikum-/Antibiotika-Zubereitung nach einem der Ansprüche 1 bis **3**, **dadurch gekennzeichnet, dass** die antibiotische Komponente in der protonierten Salzform vorliegt, wobei Chlorid-Ionen, Bromid-Ionen, Hydrogensulfat-Ionen, Sulfat-Ionen, Dihydrogenphosphat-Ionen, Hydrogenphosphat-Ionen, Phosphat-Ionen, Acetat-Ionen, Succinat-Ionen und Lactat-Ionen als Gegenionen verwendet werden.

5. Antibiotikum-/Antibiotika-Zubereitung nach einem der Ansprüche 1 bis **4**, **dadurch gekennzeichnet, dass** diese als durch Pressen und/oder Strangpressen und/oder Mahlen und/oder Kalandrieren und/oder Gießen und/oder Spinnen und /der Sintern hergestellte Formkörper, Granulate, Folien, Pulver, Schläuche, Vliese oder Fäden vorliegt.

6. Antibiotikum-/Antibiotika-Zubereitung nach einem der Ansprüche 1 bis **5**, **dadurch gekennzeichnet, dass** die amphiphile Komponente und die antibiotische Komponente in der wasserfreien, organischen Hilfskomponente suspendiert sind und eine injizierbare Suspension bilden.

7. Verwendung einer Antibiotikum-/Antibiotika-Zubereitung nach einem der Ansprüche 1 bis **6** zur Herstellung eines Implantates.

8. Verwendung einer Antibiotikum-/Antibiotika-Zubereitung nach einem der Ansprüche 1 bis **6** zur Herstellung einer injizierbaren Suspension.

9. Verwendung einer Antibiotikum-/Antibiotika-Zubereitung nach Anspruch **8** zur Herstellung eines Implantates in Form von Formkörpern, Granulaten, Pulvern, Schläuchen, Folien, Vliesen und Fäden.

10. Verwendung nach Anspruch **9**, **dadurch gekennzeichnet, dass** die aus der Antibiotikum-/Antibiotika-Zubereitung hergestellten Formkörper, Granulate, Pulver, Schläuche, Folien, Vliesen und Fäden plastisch verformbar und modellierbar sind. r

11. Verwendung einer Antibiotikum-/Antibiotika-Zubereitung nach einem der Ansprüche **1** bis **6** als Beschichtung auf resorbierbaren porösen Gläsern, auf nicht resorbierbaren Gläsern, auf resorbierbaren porösen Glaskeramiken, auf nicht resorbierbaren porösen Glaskeramiken, auf resorbierbaren porösen Keramiken und auf nicht resorbierbaren porösen Keramiken.

12. Verwendung einer Antibiotikum-/Antibiotika-Zubereitung nach einem der Ansprüche **1** bis **6** als Beschichtung auf resorbierbaren Kunststoffimplantaten, auf nicht resorbierbaren Kunststoffimplantaten und auf Metallimplantaten.

13. Verwendung einer Antibiotikum-/Antibiotika-Zubereitung nach einem der Ansprüche **1** bis **6** zur Herstellung eines Arzneimittels **dadurch gekennzeichnet, dass** durch das Verhältnis der Stoffmenge der amphiphilen Komponente zur Stoffmenge der antibiotischen Komponente der Anteil der verzögert freigesetzten antibiotischen Komponente an der Gesamtmenge der antibiotischen Komponente bestimmt wird.

14. Verfahren zur Herstellung von eine Antibiotikum-/Antibiotika-Zubereitung nach einem der Ansprüche **1** bis **6** enthaltenden Implantaten, **dadurch gekennzeichnet, dass** Implantate in Form von Formkörpem, Granulaten, Pulvern, Schläuchen, Folien, Vliesen und Fäden mit der Antibiotikum-/Antibiotika-Zubereitung nach einem der Ansprüche **1** bis **6** durch Pressen und/oder Tauchen und/oder Sprühen und/oder Kalandrieren und/oder Strangpressen und/oder Sintern und/oder Aufschmelzen beschichtet werden.

## Claims

1. A formulation comprising an antibiotic/antibiotics, **characterized by** a mixture of at least one amphiphilic component of a member of the alkylsulphates, arylsulphates, alkylarylsulphates, cycloalkylsulphates, alkylcycloalkylsulphates, alkylsulphamates, cycloalkylsulphamates, alkylcycloalkylsulphamates, arylsulphamates, alkylarylsulphamates, alkylsulphonates, fatty acid-2-sulphonates, arylsulphonates, alkylarylsulphonates, cycloalkylsulphonates, alkylcycloalkylsulphonates, alkyldisulphates, cycloalkyldisulphates, alkyldisulphonates, cycloalkyldisulphonates, aryldisulphonates, alkylaryldisulphonates, aryltrisulphonates and alkylaryltrisulphonates
- at least one antibiotic component from the group consisting of the aminoglycoside antibiotics, the lincosamide antibiotics, the 4-quinolone antibiotics-and tetracycline antibiotics and
- a) at least one inorganic auxiliary component from the groups consisting of
- calcium hydrogen phosphate, calcium hydrogen phosphate dihydrate, hydroxyapatite, fluoroapatite, calcium polyphosphate, tricalcium phosphate, tetracalcium phosphate, calcium sulphate, calcium sulphate hemihydrate, calcium sulphate dihydrate, calcium lactate, sodium bicarbonate, calcium carbonate, magnesium carbonate, calcium hydroxide, magnesium hydroxide, magnesium oxide,
- preceding substances in the form of coarsely disperse and/or highly disperse powders
- absorbable glasses, nonabsorbable glasses, absorbable glass ceramic, nonabsorbable glass ceramic, absorbable ceramic and nonabsorbable ceramic and
- b) at least one anhydrous, organic auxiliary component which comprises hydrolytically cleavable carboxylic ester bonds and/or hydrolytically cleavable carboxamide bonds and/or hydrolytically cleavable carboxylic anhydride bonds and/or hydrolytically cleavable phosphoric ester bonds and/or hydrolytically cleavable phosphoramide bonds and/or enzymatically cleavable carboxylic ester bonds and/or enzymatically cleavable carboxamide bonds and/or enzymatically cleavable carboxylic anhydride bonds and/or enzymatically cleavable phosphoric ester bonds and/or enzymatically cleavable phosphoramide bonds.

2. Formulation comprising an antibiotic/ antibiotics according to Claim 1, **characterized in that** it contains at least one biologically active auxiliary component from the group consisting of the penicillin antibiotics, the cephalosporin antibiotics, the 4-quinolone antibiotics and the macrolide antibiotics or optionally one or more members of the sulphonamide chemotherapeutic agents, the analgesics and the antiphlogistic agents.

3. Formulation comprising an antibiotic/ antibiotics according to either of Claims 1 and 2, **characterized in that** the antibiotic component contains at least one amino group.

4. Formulation comprising an antibiotic/ antibiotics according to any of Claims 1 to 3, **characterized in that** the antibiotic component is present in the protonated salt form, chloride ions, bromide ions, hydrogen sulphate ions, sulphate ions, dihydrogen phosphate ions, hydrogen phosphate ions, phosphate ions, acetate ions, succinate ions and lactate ions being used as opposite ions.

5. Formulation comprising an antibiotic/ antibiotics according to any of Claims 1 to 4, **characterized in that** it is present in the form of mouldings, granules, films, powders, tubes, nonwovens or filaments produced by pressing and/or extrusion and/or milling and/or calendering and/or casting and/or spinning and/or sintering.

6. Formulation comprising an antibiotic/ antibiotics according to any of Claims 1 to 5, **characterized in that** the amphiphilic component and the antibiotic component are suspended in the anhydrous, organic auxiliary component and form an injectable suspension.

7. Use of a formulation comprising an antibiotic/antibiotics according to any of Claims 1 to 6 for the production of an implant.

8. Use of a formulation comprising an antibiotic/ antibiotics according to any of Claims 1 to 6 for the production of an injectable suspension.

9. use of a formulation comprising an antibiotic/ antibiotics according to Claim 8 for the production of an implant in the form of mouldings, granules, powders, tubes, films, nonwovens and filaments.

10. Use according to Claim 9, **characterized in that** the mouldings, granules, powders, tubes, films, nonwovens and filaments produced from the formulation comprising an antibiotic/antibiotics are plastically deformable and capable of being modelled.

11. Use of a formulation comprising an antibiotic/ antibiotics according to any of Claims 1 to 6 as a coating on absorbable porous glasses, on nonabsorbable glasses, on absorbable porous glass ceramics, on nonabsorbable porous glass ceramics, on absorbable porous ceramics and on nonabsorbable porous ceramics.

12. Use of a formulation comprising an antibiotic/ antibiotics according to any of Claims 1 to 6 as a coating on absorbable plastic implants, on nonabsorbable plastic implants and on metal implants.

13. Use of a formulation comprising an antibiotic/ antibiotics according to any of Claims 1 to 6 for the production of a drug, **characterized in that** the proportion of the delayed-release antibiotic component, based on the total amount of the antibiotic component, is determined by the ratio of the amount of the amphiphilic component to the amount of the antibiotic component.

14. Process for the production of implants containing a formulation comprising an antibiotic/ antibiotics according to any of Claims 1 to 6, **characterized in that** implants in the form of mouldings, granules, powders, tubes, films, nonwovens and filaments are coated with the formulation comprising an antibiotic/antibiotics according to any of Claims 1 to 6 by pressing and/or immersion and/or spraying and/or calendering and/or extrusion and/or sintering and/or melting.

## Revendications

1. Préparation d'antibiotique/antibiotiques **caractérisée par** un mélange
- d'au moins un composant amphiphile d'un représentant des alkylsulfates, arylsulfates, alkylarylsulfates, cycloalkylsulfates, alkylcycloalkylsulfates, alkylsulfamates, cycloalkylsulfamates, alkylcycloalkylsulfamates, arylsulfamates, alkylarylsulfamates, alkylsulfonates, acide gras-2-sulfonates, arylsulfonates, alkylarylsulfonates, cycloalkylsulfonates, alkylcycloalkylsulfonates, alkyldisulfates, cycloalkyldisulfates, alkyldisulfonates, cycloalkyldisulfonates, aryldisulfonates, alkylaryldisulfonates, aryltrisulfonates et alkylaryltrisulfonates,
- d'au moins un composant antibiotique du groupe des antibiotiques de type aminoglycoside, des antibiotiques de type lincosamide, des antibiotiques de type 4-quinolone et des antibiotiques de type tétracycline ainsi que
- a) au moins un composant auxiliaire inorganique des groupes
- hydrogénophosphate de calcium, hydrogénophosphate de calcium dihydraté, hydroxylapatite, fluoroapatite, polyphosphate de calcium, phosphate de tricalcium, phosphate de tétracalcium, sulfate de calcium, sulfate de calcium hémihydraté, sulfate de calcium dihydraté, lactate de calcium, hydrogénocarbonate de calcium, carbonate de calcium, carbonate de magnésium, hydroxyde de calcium, hydroxyde de magnésium, oxyde de magnésium,
- les substances précédentes sous forme de poudre dispersée grossièrement et/ou hautement dispersée,
- les verres résorbables, les verres non résorbables, les vitro-céramiques résorbables, les vitrocéramiques non résorbables, les céramiques résorbables et les céramiques non résorbables, et
- b) au moins un composant auxiliaire organique anhydre qui comporte des liaisons ester d'acide carboxylique clivables par hydrolyse et/ou des liaisons amide d'acide carboxylique clivables par l'hydrolyse et/ou des liaisons anhydride d'acide carboxylique clivables par hydrolyse et/ou des liaisons ester d'acide phosphorique clivables par hydrolyse et/ou des liaisons amide d'acide phosphorique clivables par hydrolyse et/ou des liaisons ester d'acide carboxylique clivables par voie enzymatique et/ou des liaisons amide d'acide carboxylique clivables par voie enzymatique et/ou des liaisons anhydride d'acide carboxylique clivables par voie enzymatique et/ou des liaisons ester d'acide phosphorique clivables par voie enzymatique et/ou des liaisons amide d'acide phosphorique clivables par voie enzymatique.

2. Préparation d'antibiotique/antibiotiques selon la revendication 1 **caractérisée en ce que** celle-ci contient au moins un composant auxiliaire biologiquement actif du groupe des antibiotiques de type pénicilline, des antibiotiques de type céphalosporine, des antibiotiques de type 4-quinolone et des antibiotiques de type macrolide ou bien éventuellement un ou plusieurs représentants des agents chimiothérapeutiques de type sulfonamide, des analgésiques et des antiphlogistiques.

3. Préparation d'antibiotique/antibiotiques selon l'une des revendications 1 à 2 **caractérisée en ce que** le composant antibiotique contient au moins un groupe amino.

4. Préparation d'antibiotique/antibiotiques selon l'une des revendications 1 à 3 **caractérisée en ce que** le composant antibiotique est présent sous forme saline protonée, où des ions chlorure, des ions bromure, des ions hydrogénosulfate, des ions sulfate, des ions dihydrogénophosphate, des ions hydrogénosphosphate, des ions phosphate, des ions acétate, des ions succinate et des ions lactate sont utilisés comme contre-ions.

5. Préparation d'antibiotique/antibiotiques selon l'une des revendications 1 à 4 **caractérisée en ce que** celle-ci est présente sous forme de corps mis en forme, de granulés, de feuilles, de poudres, de tubes, de nappes ou de fils produits par compression et/ou extrusion et/ou broyage et/ou calandrage et/ou coulée et/ou filage et/ou frittage.

6. Préparation d'antibiotique/antibiotiques selon l'une des revendications 1 à 5 **caractérisée en ce que** le composant amphiphile et le composant antibiotique sont en suspension dans le composant auxiliaire organique anhydre et forment une suspension injectable.

7. Utilisation d'une préparation d'antibiotique/antibiotiques selon l'une des revendications 1 à 6 pour la production d'un implant.

8. Utilisation d'une préparation d'antibiotique/antibiotiques selon l'une des revendications 1 à 6 pour la production d'une suspension injectable.

9. Utilisation d'une préparation d'antibiotique/antibiotiques selon la revendication 8 pour la production d'un implant sous forme de corps mis en forme, de granulés, de poudres, de tubes, de feuilles, de nappes et de fils.

10. Utilisation selon la revendication 9 **caractérisée en ce que** les corps mis en forme, granulés, poudres, tubes, feuilles, nappes et fils produits à partir de la préparation d'antibiotique/antibiotiques peuvent être déformés et façonnés plastiquement.

11. Utilisation d'une préparation d'antibiotique/antibiotiques selon l'une des revendications 1 à 6 comme revêtement sur des verres poreux résorbables, sur des verres non résorbables, sur des vitrocéramiques poreuses résorbables, sur des vitrocéramiques poreuses non résorbables, sur des céramiques poreuses résorbables et sur des céramiques poreuses non résorbables.

12. Utilisation d'une préparation d'antibiotique/antibiotiques selon l'une des revendications 1 à 6 comme revêtement sur des implants en matière plastique résorbables, sur des implants en matière plastique non résorbables et sur des implants métalliques.

13. Utilisation d'une préparation d'antibiotique/antibiotiques selon l'une des revendications 1 à 6 pour la production d'un médicament **caractérisée en ce que** la proportion de composant antibiotique libéré de manière retardée par rapport à la quantité totale de composant antibiotique est déterminée par le rapport de la quantité de substance du composant amphiphile à la quantité de substance du composant antibiotique.

14. Procédé de production d'implants contenant une préparation d'antibiotique/antibiotiques selon l'une des revendications 1 à 6 **caractérisé en ce que** des implants sous forme de corps mis en forme, de granulés, de poudres, de tubes, de feuilles, de nappes et de fils sont revêtus de la préparation d'antibiotique/antibiotiques selon l'une des revendications 1 à 6 par compression et/ou immersion et/ou pulvérisation et/ou calandrage et/ou extrusion et/ou frittage et/ou application en fusion.
